# EUROPEAN PATENT APPLICATION

(11) **EP 2 286 779 A1**
(43) Date of publication of application: **23.02.2011**
(21) Application number: 10181353.3
(22) Date of filing: 19.08.2002
(51) Int. Cl.: A61F 13/496, A61F 13/539

(54) **Pants-type disposable diaper**

(30) Priority: 23.08.2001 JP 2001252594
(62) Divisional of application: 02255771.4
(71) Applicant: Uni-Charm Corporation, Ehime (JP)
(72) Inventor: Ishikawa, Hiroki, Mitoyo-gun Kagawa 769-1602 (JP); Kinoshita, Akiyoshi, Mitoyo-gun Kagawa 769-1602 (JP)
(74) Representative: Kensett, John Hinton

(57) **Abstract**

A pants-type disposable diaper 1A basically comprises pants 2 and a liquid-absorbent pad 3 laid on an inner surface of the pants 2. The pad 3 is contoured by longitudinally opposite end portions 3a bonded to the pants 2 in the vicinity of waist-hole's peripheral edge portions 9 of the pants 2 and transversely opposite side edge portions 3b extending across a crotch region 7 of the pants 2 toward the waist-hole's peripheral edge portions 9 in a longitudinal direction. First and second stretchable elastic members 23, 24 extending in the longitudinal direction are bonded in a stretched state to an outer surface of a backsheet 19 forms the pad 3 so that the area of the pad 3 defined between the longitudinally opposite end portions 3a thereof is lifted off from the inner surface of the pants as these elastic members 23, 24 contact.

## Description

This invention relates to a pants-type disposable diaper for absorption and containment of bodily discharges.

Japanese Patent Publication No. 1997-56747A discloses a pants-type disposable diaper comprising pants composed of front and rear waist regions and a crotch region extending between these waist regions so as to define a waist-hole and a pair of leg-holes below the waist-hole and a liquid-absorbent pad laid on an inner surface of the pants and extending across the crotch region into the front and rear waist regions of the pants. The pad comprises a liquid-pervious topsheet facing the wearer's body, a liquid-impervious backsheet facing a garment the wearer put on and a liquid-absorbent core interposed between these top- and backsheets. The pad has longitudinally opposite end portions lying on waist-hole's peripheral edge portions of the pants and extending in a waist-surrounding direction and a middle portion laid on the crotch region of the pants. The pad is bonded to the pants at these longitudinally opposite end portions and the middle portion while a zone of the pad defined between the longitudinally opposite end portions and the middle portion in free from the pants.

In this diaper of well known art, the liquid-absorbent pad is bonded to the pants at the waist-hole's peripheral edge portions and the crotch region of the pants and therefore it is not likely that the pad might be lifted off from the crotch region of the pants and/or shift sideways. Consequently, the pants can be easily worn without any hindrance. In addition, neither stretching nor contraction of the pants in the waist-surrounding direction is obstructed by the presence of the pad because a zone of the pad defined between the longitudinally opposite end portions and the middle portions is free from the pants.

In the case of the diaper disclosed by the above-cited Publication, the middle portion of the pad is joined to the crotch region of the pants and therefore it is sometimes difficult to keep the pad in close contact with the wearer's body since the pad may slip down as the pants slip down. Additionally, while the zone of the pad defined between the longitudinally opposite end portions and the middle portion is free from the pants, this diaper of well known art has no means by which this zone of the pad is actively put in close contact with the wearer's body.

An object of this invention is to provide a pants-type disposable diaper improved so that a pad can be reliably put in close contact with the wearer's body and kept in such a close contact even if the pants slip down.

According to this invention, there is provided a pants-type disposable diaper comprising pants composed of front and rear waist regions opposed to each other and a crotch region extending between these waist regions so as to define a waist-hole and a pair of leg-holes below the waist-hole and a liquid-absorbent pad laid on an inner surface of the pants and extending across the crotch region toward the front and rear waist regions of the pants. The pants are contoured by longitudinally opposite end portions bonded to the front and rear waist regions of the pants so as to extend across the front and rear waist regions in a waist-surrounding direction and transversely opposite side edge portions extending across the crotch region toward the front and rear waist regions of the pants in a longitudinal direction.

This invention further comprises stretchable elastic members extending in the longitudinal direction in parallel to the transversely opposite side edge portions of the pad and bonded to an opposed surface of the pad facing the pants in a stretched state so that an area of the pad defined between the longitudinally opposite end portions thereof is lifted off from the inner surface of the pants as the elastic members contract.

This invention includes the following preferred embodiments, the elastic members comprise at least one first elastic member attached to the pad in the vicinity of a longitudinal center line bisecting a dimension between the transversely opposite side edge portions of the pad and at least one second elastic members attached to the pad so as to lie between the longitudinal center line and each of the transversely opposite side edge portions of the pad and wherein each of the first and second elastic members comprises longitudinally opposite end portions lying on the longitudinally opposite end portions of the pad and a longitudinally intermediate portion defined between the longitudinally opposite end portions.

The longitudinally intermediate portions of the second elastic members curve in the crotch region of the pants so as to describe substantially circular arcs which are convex toward the longitudinal center line so that a distance between the first and second elastic members is relatively small in the crotch region of the pants and relatively large in the front and rear waist regions of the pants.

Waist-surrounding elastic members extending in the waist-surrounding direction are bonded in a stretched state to waist-hole's peripheral edge portions of the pants and the longitudinally opposite end portions of the pad are bonded to the pants at a lower portion of the waist-surrounding elastic members.

The transversely opposite side edge portions of the pad are bonded to the crotch region of the pants.

The pad comprises a liquid-pervious topsheet facing the wearer's body, a liquid-impervious backsheet facing a garment the wearer puts on and a liquid-absorbent core interposed between the top- and backsheets and wherein the stretchable elastic members are attached to the backsheet.
Fig. 1 is a perspective view of a disposable diaper;
Fig. 2 is a perspective view showing the diaper of Fig. 1 before shaped in pants-type;
Fig. 3 is a sectional view taken along a line A - A in Fig. 1;
Fig. 4 is a cross-sectional view taken along a line B - B in Fig. 1;
Fig. 5 is a perspective view of another preferred embodiment of the diaper according to this invention;
Fig. 6 is a perspective view showing the diaper of Fig. 5 before shaped in pants-type; and
Fig. 7 is a cross-sectional view taken along a line C - C in Fig. 5.

Details of a pants-type disposable diaper will be more fully understood from the description given hereunder in reference to the accompanying drawings.

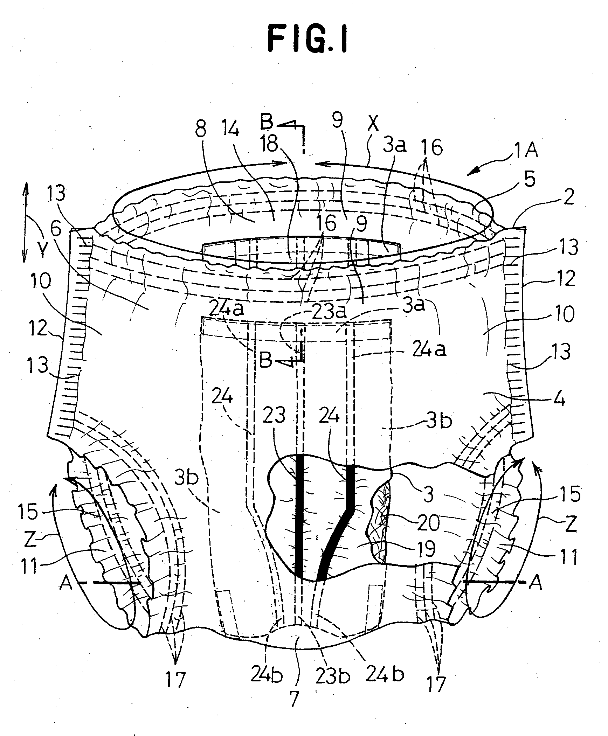

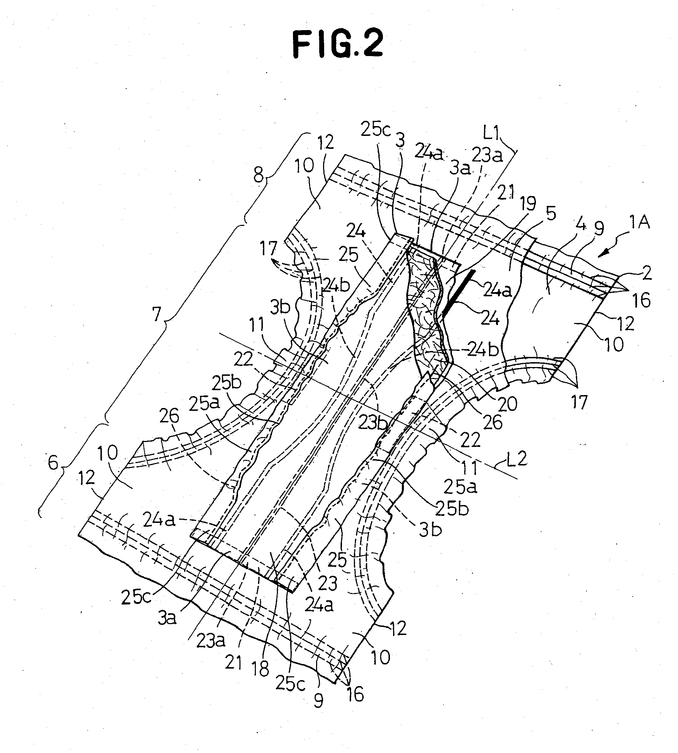

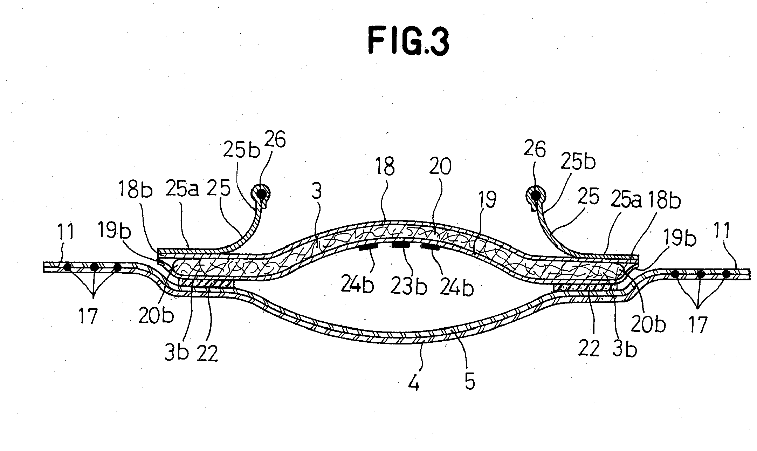

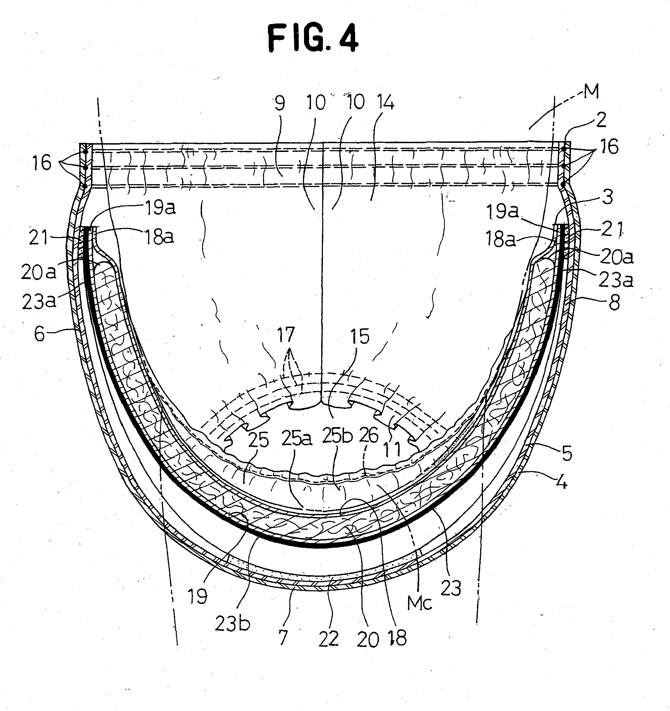

Fig. 1 is a perspective view of a disposable diaper 1A, Fig. 2 is a perspective view showing the diaper 1A of Fig. 1 as before shaped in pants-type, Fig. 3 is a sectional view taken along a line A - A in Fig. 1 and Fig. 4 is a cross-sectional view taken along a line B - B in Fig. 1. In Fig. 1, a waist-surrounding direction is indicated by an arrow X, a longitudinal direction is indicated by an arrow Y and a thigh-surrounding direction is indicated by an arrow Z. In Fig. 4, a diaper wearer M is indicated by chain double-dashed lines. Expression "inner surfaces" of top- and backsheets 18, 19 should be understood to mean the surfaces thereof facing a core 20 and expression "outer surfaces" of these sheets 18, 19 should be understood to mean the surfaces thereof facing away from the core 20.

The diaper 1A comprises pants 2 and a liquid-absorbent pad 3 attached to the inner surface of the pants 2.

The pants 2 comprise substantially liquid-impervious outer and inner sheets 4, 5 overlaid each other. These outer and inner sheets 4, 5 have surfaces facing and joined together. The pants 2 are composed of front and rear waist regions 6, 8 opposed to each other and a crotch region 7 lying between these the waist regions 6, 8.

The pants 2 have waist-hole's peripheral edge portions 9 extending in the front and rear waist regions 6, 8 in the waist-surrounding direction, waist's side portions 10 extending in the front and rear waist regions 6, 8 in the longitudinal direction and leg-holes' peripheral edge portions 11 extending in the crotch region 7 in the thigh-surrounding direction. The front and rear waist regions 6, 8 are overlaid and joined together in the vicinity of side edges 12 contiguous to the waist's side portions 10 by means of a plurality of heat-sealing zones 13 arranged in the vicinity of the side edges 12 intermittently in the longitudinal direction. The pants 2 are formed with a waist-hole 14 and a pair of leg-holes 15 lying below the waist-hole 14.

The waist-hole's peripheral edge portions 9 are provided with a plurality of waist-surround elastic members 16 extending in the waist-surrounding direction and attached thereto in a stretched state. The leg-holes' peripheral edge portions 15 are provided with a plurality of thigh-surrounding elastic members 17 extending in the thigh-surrounding direction and attached thereto in a stretched state. Both the waist-surrounding elastic members 16 and the thigh-surrounding elastic members 17 are interposed between the outer sheet 4 and the inner sheet 5 and bonded to opposed surfaces of these sheets 4, 5.

The pad 3 basically comprises a liquid-pervious topsheet 18 facing the wearer's body, a liquid-impervious backsheet 19 facing a garment the wearer puts on and a liquid-absorbent core 20 interposed between these top- and backsheets 18, 19. The core 20 is bonded to inner surfaces of the top- and backsheets 18, 19. The pad 3 is substantially rectangular in its planar shape and extends across the crotch region 7 into the front and rear waist regions 6, 8 of the pants 2 in the longitudinal direction. The pad 3 curves substantially in U-shape so as to follow the curve of the pants 2 in the crotch region 7.

The pad 3 of the pants 2 is contoured by longitudinally opposite end portions 3a lying in the vicinity of the waist-hole's peripheral edge portions 9 and extending in the waist-surrounding direction and transversely opposite side edge portions 3b extending across the crotch region 7 toward the waist-hole' s peripheral edge portions 9 in the longitudinal direction. The pad 3 has its longitudinally opposite end portions 3a bonded to the pants 2 in the vicinity of the waist-hole's peripheral edge portions 9 by means of a hot melt adhesive 21 and its transversely opposite side edge portions 3b bonded to the crotch region 7 of the pants 2 by means of a hot melt adhesive 22. Except for the longitudinally opposite end portions 3a and the transversely opposite side edge portions 3b extending in the crotch region 7, the pad 3 is not joined to the inner sheet 5 and therefore free from the pants 2. In the longitudinally opposite end portions 3a and the transversely opposite side edge portions 3b of the pad 3, the backsheet 19 forming the pad 3 has its outer surface bonded to the inner sheet 5.

Along the longitudinally opposite end portions 3a of the pad 3, respective end portions 18a, 19a of the top- and backsheets 18, 19 extending outward beyond longitudinally opposite ends 20a of the core 20 are overlaid and inner surfaces of these sheets 18, 19 are joined together at these end portions 18a, 19a. Along the transversely opposite side edge portions 3b of the pad 3, respective side edge portions 18b, 19b of the top- and backsheets 18, 19 extending outward beyond transversely opposite side edges 20b of the core 20 are overlaid upon each other and inner surfaces of these sheets 18, 19 are joined together at these side edge portions 18b, 19b.

The pad 3 is provided with first stretchable elastic member 23 and second stretchable elastic members 24 extending in parallel to the transversely opposite side edge portions 3b across the crotch region 7 toward the waist-hole's peripheral edge portions 9 of the pants 2 and attached thereto in a stretched state. The pad 3 is provided along its side edge portions 3b with lateral sheets 25 which are substantially liquid-impervious and extend across the crotch region 7 toward the waist-hole's peripheral edge portions 9 of the pants 2. In the diaper 1A, the longitudinally opposite end portions 3a and the transversely opposite side edge portions 3b of the pad 3 are bonded to the pants 2 with the first and second elastic members 23, 24 attached to the pad 3 being stretched in the longitudinal direction.

As seen in Fig. 2, the first elastic member 23 lies on a longitudinal center line L1 bisecting a dimension between the transversely opposite side edge portions 3b of the pad 3. The second elastic members 24 lie between the longitudinal center line L1 and the respective side edge portions 3b of the pad 3. The first elastic member 23 and the second elastic members 24 are bonded to the outer surface of the backsheet 19. These first and second elastic members 23, 24 are not bonded to the inner sheet 5 and therefore free from the pants 2.

Both the first elastic member 23 and the second elastic members 24 are band-like and respectively comprise longitudinally opposite end portions 23a, 24a lying on the longitudinally opposite end portions 3a and longitudinally intermediate portions 23b, 24b extending between the longitudinally opposite end portions 23a, 24a, respectively. The first elastic member 23 extends on the longitudinal center line L1 almost linearly. The second elastic members 24 respectively have the longitudinally intermediate portions 24b curving so as to describe substantially circular arcs which are convex toward the longitudinal center line L1 in the crotch region 7 of the pants 2. Distance between the first elastic member 23 and the second elastic members 24 is relatively small in the crotch region 7 of the pants 2 and relatively large in the front and rear waist regions 6, 8 of the pants 2.

Each of the lateral sheets 25 has a fixed side edge portion 25a fixed to the associated side edge portion 3b of the pad 3 and extending in the longitudinal direction, a free side edge portion 25b extending in parallel to the fixed side edge portion 25a in the longitudinal direction and longitudinally opposite fixed end portions 25c collapsed inward in the transverse direction of the pad 3 and fixed to the associated end portion 3a of the pad 3 in such a collapsed state. The fixed side edge portion 25a and the fixed end portion 25c are bonded to the outer surface of the topsheet 18. The free side edge portion 25b is provided with a stretchable elastic member 26 extending in the longitudinal direction and attached thereto in a stretched state. The elastic member 26 is wrapped with a part of the associated free side edge portion 25b.

To shape the diaper 1A of Fig. 2 in pants-type, the pants 2 may folded along a transverse center line L2 extending across the crotch region 7 of the pants 2 with the outer surface of the topsheet 18 inside being opposed to each other and then the front and rear waist regions 6, 8 may be connected to each other by bonding in the vicinity of the side edges 12 contiguous to the respective waist's side portions 10 of the pants 2.

In the diaper 1A, contraction of the first and second elastic members 23, 24 in the longitudinal direction causes the pad 3 except for the longitudinally opposite end portions 3a and the transversely opposite side edge portions 3b extending in the crotch region 7 to be lifted off from the inner surface of the pants 2. In the diaper 1A, the pad 3 can be reliably kept in close contact with the body of the wearer M of the diaper 1A even if the pants 2 slip down.

In the diaper 1A, the longitudinally intermediate portions 24b of the second elastic members 24 curve so as to describe the substantially circular arcs which are convex toward the longitudinal center line L1 and, in the crotch region 7 of the pants 2, the pad 3 is lifted off, in the vicinity of the longitudinal center line L1, from the inner surface of the pants 2. In other words, the pad 3 is formed into an inverted V-shape in the crotch region 7 of the pants 2 (See Fig. 3). Such a form allows the pad 3 to be fitted to a crotch Mc of the wearer M in the crotch region 7 and thereby alleviate any feeling of discomfort against the wearer M when the pad 3 is put in contact with the crotch Mc of the wearer M.

In the diaper 1A, the longitudinally opposite end portions 3a of the pad 3 is bonded to the pants 2 at the lower portion of the waist-surrounding elastic members 16, so it is not likely that the longitudinally opposite end portions 9 of the pants 2 might slip down even if the first and second elastic members 23, 24 contract in the longitudinal direction. The diaper 1A is also free from inconvenience that the longitudinally opposite end portions 3a of the pad 3 might obstruct a contraction of the waist-surrounding elastic members 16, so the torso of the wearer M can be reliably tightened by using the waist-surrounding elastic members 16.

In the diaper 1A, the transversely opposite side edge portions 3b of the pad 3 extending across the crotch region 7 of the pants 2 are bonded to the pants 2. Such an arrangement is effective to prevent the pad 3 from shifting sideways in the crotch region 7 of the pants 2 and ensures the diaper 1A to be worn without any hindrance.

In the diaper 1A, the free side edge portions 25b of the lateral sheets 25 attached to the pad 3 rise on the topsheet 18 as the elastic members 26 contract in the longitudinal direction. These free side edge portions 25b of the lateral sheets 25 form barriers against bodily discharges so as to prevent bodily discharges from leaking sideways beyond the transversely opposite side edge portions 3b of the pad 3.

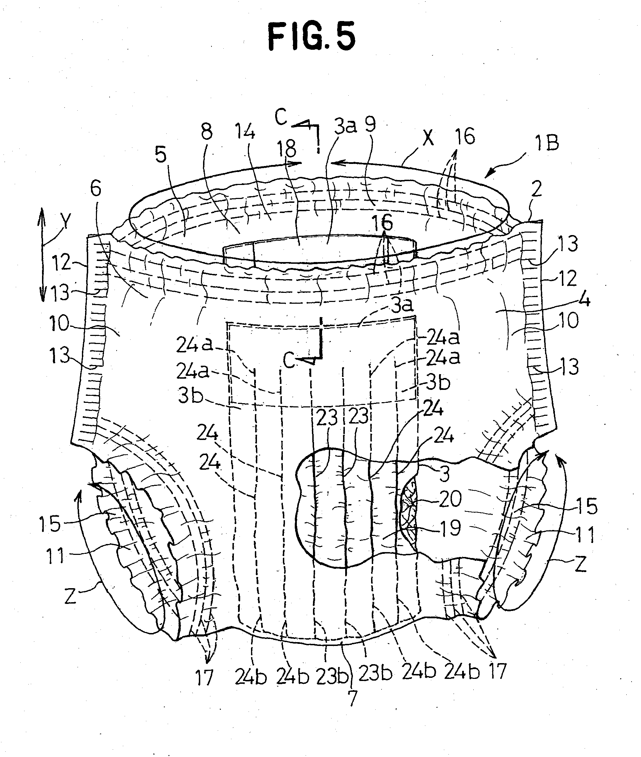

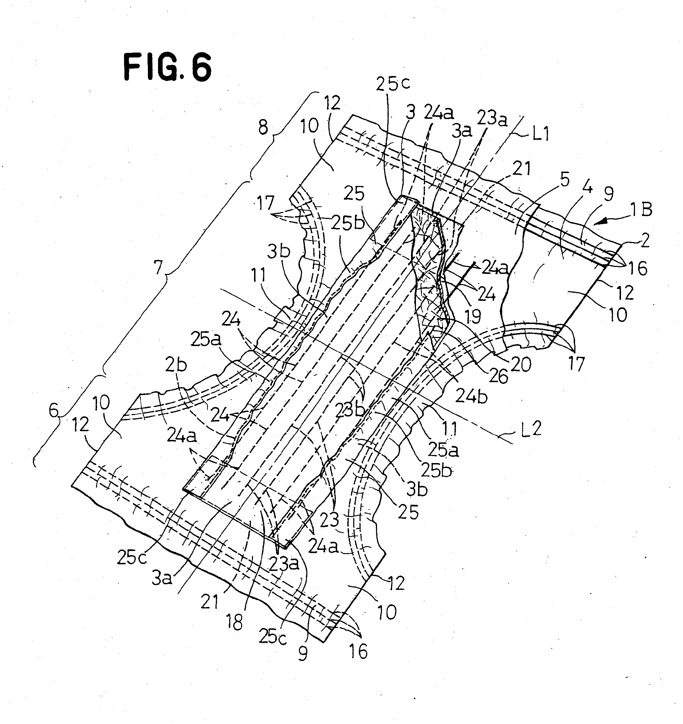

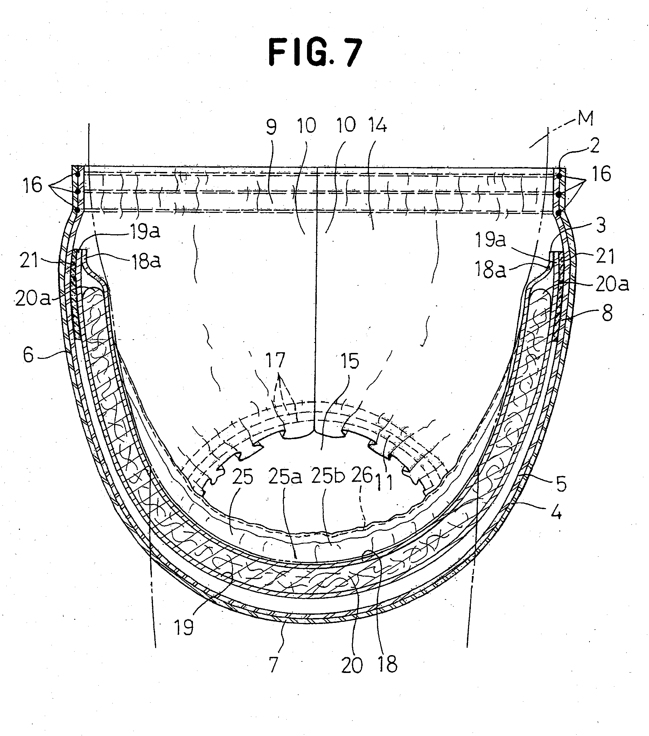

Fig. 5 is a perspective view of another embodiment 1B of the disposable diaper according to this invention, Fig. 6 is a perspective view showing the diaper 1B of Fig. 5 as before shaped in pants-type and Fig. 7 is a cross-sectional view taken along a line C - C in Fig. 5. In Fig. 5, the waist-surrounding direction is indicated by an arrow X, the longitudinal direction is indicated by an arrow Y and the thigh-surrounding direction is indicated by an arrow Z. In Fig. 7, the wearer M is indicated by chain double-dashed lines.

There are differences between the diaper 1B and the diaper of Fig. 1 as follows. In the diaper 1B, the pad 3 has its longitudinally opposite end portions 3a bonded to the pants 2 in the vicinity of the waist-hole's peripheral edge portions 9 by means of a hot melt adhesive 21 and the other area of the pad 3 is free from the pants 2. The longitudinally opposite end portions 3a of the pad 3 are bonded to the pants 2 at the lower portion of the waist-surrounding elastic members 16. The pad 3 is provided with a plurality of the first stretchable elastic members 23 and the second stretchable elastic members 24 extending in the longitudinal direction in parallel to the transversely opposite side edge portions 3b across the crotch region 7 into the front and rear waist regions 6, 8 and attached thereto in a stretched state.

The first elastic members 23 are thread-like and lie on both sides of the longitudinal center line L1. The first elastic members 23 extend almost linearly between the longitudinally opposite end portions 3a of the pad 3. The second elastic members 24 are also thread-like and lie between the longitudinal center line L1 and the respective side edge portions 3b. The second elastic members 24 respectively have the longitudinally intermediate portions 24b extending almost linearly between the longitudinally opposite end portions 3a of the pad 3 without curving so as to describe substantially circular arcs which are convex toward the longitudinal center line L1. Both of the first and second elastic members 23, 24 respectively have the longitudinally opposite end portions 23a, 24a lying at the lower portion of the longitudinally opposite end portions 3a of the pad 3. Distance between the first elastic members 23 and the second elastic members 24 is approximately uniform in the front and rear waist regions 6, 8 as well as in the crotch region 7. In the case of the diaper 1B, the longitudinally opposite end portions 3a of the pad 3 are bonded to the pants 2 with the first and second elastic members 23, 24 attached to the pad 3 being stretched in the longitudinal direction.

In the diaper 1B, contraction of the first and second elastic members 23, 24 in the longitudinal direction causes the pad 3 except for the longitudinally opposite end portions 3a to be lifted off from the inner surface of the pants 2. In the diaper 1B, the pad 3 is free from the crotch region 7 of the pants 2 and therefore a substantially entire area of the pad 3 is lifted off from the inner surface of the pants 2. In this way, the substantially entire area of the pad 3 can be put in close contact with the body of the wearer M. In the diaper 1B, the pad 3 can be reliably kept close contact with the body of the wearer M even if the pants 2 slip down.

For these illustrated embodiments 1A, 1B of the diaper, the first and second elastic members 23, 24 are bonded to the backsheet 19 of the pad 3 preferably using a hot melt adhesive. Bonding of these first and second elastic members 23, 24 using the hot melt adhesive may be carried out selectively in an appropriate adhesive coating pattern such as a spiral pattern or a spray pattern. An adhesive coated area percentage per total surface area of the first and second elastic members 23, 24 is preferably in a range of 20 - 80% in order that these first and second elastic members 23, 24 can be reliably bonded to the backsheet 19. If the adhesive coated area percentage is less than 20%, there would be an anxiety that these elastic members 23, 24 might be torn off from the backsheet 19. If the adhesive coated area percentage exceeds 80%, the desired contraction of these elastic members 23, 24 would be obstructed by the presence of the adhesive.

In these illustrated embodiments 1A, 1B of the diaper, it is essential that the first and second elastic members 23, 24 extend across the crotch region 7 of the pants 2 into the front and rear waist regions 6, 8 in order that the pad 3 except for its longitudinally opposite end portions 3a is reliably lifted off from the inner surface of the pants 2. However, it is unnecessary that the first and second elastic members 23, 24 should extend to the waist-hole's peripheral edge portions 9 of the pants 2 so far as the longitudinally opposite ends 23a, 24a of these elastic members 23, 24 extend to respective zones of the front and rear waist regions 6, 8 immediately adjacent the crotch region 7.

In these illustrated embodiments 1A, 1B of the diaper, it is also possible without departing from the scope of this invention to bond the first and second elastic members 23, 24 to the inner surface of the backsheet 19. The first and second elastic members 23, 24, the waist-surrounding elastic members 16, the thigh-surrounding elastic members 17 and the elastic members 26 may be formed by natural rubber or synthetic rubber.

Stock material for the topsheet 18 may be selected from a group including a hydrophilically modified fibrous nonwoven fabric, a thermoplastic film having a plurality of fine pores and a hydrophobic fibrous nonwoven fabric. The inner and outer sheets 5, 4 as well as the backsheet 19 may be formed using a material selected from a group including a hydrophobic fibrous nonwoven fabric, a breathable but liquid-impervious plastic film, a composite nonwoven fabric consisting of two or more layers of hydrophobic fibrous nonwoven fabrics laminated one with another and a composite sheet consisting of a hydrophobic nonwoven fabric and a breathable but liquid-impervious plastic film.

Nonwoven fabric may be selected from a group including products obtained by spun lacing, needle punching, melt blowing, thermal bonding, spun bonding, chemical bonding and air-through processes. Component fibers of the nonwoven fabric may be selected from a group including polyolefine-, polyester- and polyamide-based fibers and core-sheath-type and side-by-side-type conjugated fibers of polyethylene/polypropylene and polyethylene/polyester.

The core 20 is formed by a mixture of fluff pulp and super-absorbent polymer particles or a mixture of fluff pulp, super-absorbent polymer particles and thermoplastic synthetic resin fiber and compressed to a desired thickness. Preferably, the core 20 is entirely covered with a liquid-pervious sheet, for example, a tissue paper or a hydrophilic fibrous nonwoven fabric in order to prevent the core 20 from getting out of shape and/or to prevent the polymer particles from falling off. The polymer particles may be selected from a group consisting of a starch-based polymer, a cellulose-based polymer or a synthetic polymer.

Bonding of sheets 4, 5, 19, 18, 25, bonding of the core 20 and bonding of the elastic members 16, 17, 26 may be carried out by using a hot melt adhesive or a welding technique such as a heat-sealing and a sonic-sealing.

The pants-type disposable diaper according to this invention is primarily **characterized in that** a contraction of the stretchable elastic material in the longitudinal direction causes the pad except for the longitudinally opposite end portions thereof to be lifted off from the inner surface of the pants. Therefore the substantially entire area of the pad can be put in close contact with the body of the wearer and be kept in such a close contact with the body of the wearer even if the pants slip down.

In the case of the embodiment in which the elastic members comprise the first and second elastic members wherein the longitudinally intermediate portions of the second elastic members curve toward the longitudinal center line so as to describe the substantially circular arcs, the area of the pad extending in the crotch region is lifted off from the inner surface of the pants in the vicinity of the longitudinal center line so as to be formed into an inverted V-shape. With such a embodiment of the diaper, not only the pad can be put in close contact with the body of the wearer but also the area of the pad extending in the crotch region can be fitted with the crotch of the wearer and thereby any feeling of discomfort against the wearer when the pad contacts the crotch of the wearer is alleviated.

In the embodiment of the diaper arranged so that the longitudinally opposite end portions of the pad are bonded to the pants at the lower portion of the waist-surrounding elastic members attached to the pants, it is not likely that the waist-hole's peripheral edge portions of the pants might slip down even when the first and second elastic members contract in the longitudinal direction. With this embodiment of the diaper, a contraction of the waist-surrounding elastic members is not obstructed by the presence of the pad and the wearer's torso can be reliably tightened by using the waist-surrounding elastic members.

With the embodiment of the diaper in which the transversely opposite side edge portions of the pad is bonded to the crotch region of the pants, there is no anxiety that the pad might shift sideways in the crotch region of the pants and therefore the diaper can be worn without any hindrance.

## Claims

1. Pants-type disposable diaper(1B) comprises pants(2) composed of front and rear waist regions (6, 8) opposed to each other and a crotch region(7) extending between said waist regions so as to define a waist-hole(14) and a pair of leg-holes(15)below said waist-hole and a liquid-absorbent pad(3) laid on an inner surface of said pants and extending across said crotch region toward said front and rear waist regions of said pants wherein said pad is contoured by longitudinally opposite end portions(3a) bonded to said front and rear waist regions of said pants so as to extend across said front and rear waist regions in a waist-surrounding direction and transversely opposite side edge portions(3b) extending across said crotch region toward said front and rear waist regions of said pants in a longitudinal direction, said pants-type disposable diaper further comprising:
a plurality of elastic members(23,24)extending in said longitudinal direction in parallel to a longitudinal center line(L1) bisecting a dimension between said transversely opposite side edge portions of pad at regular intervals in a transverse direction are bonded to an opposite surface of said pad facing said pants in a stretched state.

2. The diaper according to Claim 1, wherein said elastic members (23,24)comprise at least one first elastic member (23) attached to said pad in the vicinity of said longitudinal center line and at least one second elastic member(24) each attached to said pad so as to lie between said longitudinal center line and each of said transversely opposite side edge portions of said pad and wherein each of said first and second elastic members comprises longitudinally opposite end portions lying on said pad and a longitudinally intermediate portion define between said longitudinally opposite end portions.

3. The diaper according to Claim 1, wherein waist surrounding elastic members (16) extending in said waist surrounding direction are bonded in a stretched state to waist-hole's peripheral edge portions of said pants and said longitudinally opposite end portions of said pad are bonded to said pants at a lower portion of said waist-surrounding elastic members.

4. The diaper according to Claim 1, wherein said transversely opposite side edge portions of said pad are bonded to said crotch region of said pants.

5. The diaper according to Claim 1, wherein said pad comprising a liquid pervious sheet(18) facing the wearer's body a liquid-impervious backsheet(19) facing a garment the wearer puts on and aliquid-absorbent core (20) interposed between said top-back sheets.

6. The diaper according to Claim 1, wherein said elastic members extend between vicinities of said longitudinally opposite end portions of said pad.
